# EUROPEAN PATENT SPECIFICATION

(11) **EP 3 244 961 B1**
(45) Date of publication and mention of the grant of the patent: **11.05.2022**
(21) Application number: 16737818.1
(22) Date of filing: 13.01.2016
(51) Int. Cl.: A61M 25/10, A61M 25/00

(54) **BALLOON CATHETER HAVING A RETRACTABLE SHEATH AND LOCKING MECHANISM**
BALLONKATHETER MIT EINER EINZIEHBAREN HÜLLE UND VERRIEGELUNGSMECHANISMUS
CATHÉTHER À BALLONNET COMPORTANT UNE GAINE RÉTRACTABLE ET UN MÉCANISME DE VERROUILLAGE

(30) Priority: 13.01.2015 US 201562102770 P
(43) Date of publication of application: 22.11.2017
(73) Proprietor: Pigott, John, P., Sylvania, OH 43560 (US)
(72) Inventor: Pigott, John, P., Sylvania, OH 43560 (US)
(74) Representative: Haseltine Lake Kempner LLP
(86) International application number: PCT/US2016/013251
(87) International publication number: WO 2016/115252

(56) References cited:
- EP-A1- 0 727 194
- WO-A1-2014/142801
- US-A- 5 514 093
- US-A- 5 697 948
- US-A- 5 961 536
- US-A1- 2005 149 159
- US-A1- 2007 156 225
- US-A1- 2009 105 686
- US-A1- 2013 253 467
- US-B1- 6 695 863

## Description

This application claims the benefit of U.S. Provisional Application No. 62/102,770, filed January 13, 2015.

### TECHNICAL FIELD

Exemplary embodiments of the present invention generally relate to balloon catheters used in surgical procedures.

### BACKGROUND AND SUMMARY OF THE INVENTION

This invention relates in general to balloon catheters that are used in surgical procedures. In particular, this invention relates to an improved structure for such a balloon catheter having a retractable sheath that can quickly and easily adjust the length of the inflated portion of the balloon and a locking mechanism for selectively retaining the sheath in the desired position for use.

In many surgical procedures, such as but not limited to percutaneous transluminal angioplasty procedures, a catheter having a selectively inflatable portion provided thereon (commonly referred to as a balloon catheter) is used to open a blockage and/or place a stent in a blood vessel. To accomplish this, a user must select one of various sized balloons to match the blood vessel structure and the length of the treatment area. The balloon of the catheter is initially positioned at a desired zone of attention within the blood vessel. Then, the balloon of the catheter is inflated so as to expand into engagement with an inner surface of the blood vessel, thereby expanding the blockage. If desired, an expandable stent can be disposed about the balloon of the catheter such that when the balloon is inflated, the stent is expanded into engagement with the inner surface of the blood vessel. The balloon and/or the stent is often treated with medication that is delivered by contact with the surface of the blood vessel when the balloon and/or stent is deployed. In either event, the balloon is subsequently deflated after use.

It is important that the balloon be properly sized to the treatment area. If a balloon catheter is too long, the balloon may damage the surrounding tissue. If a balloon is too short, the treatment may be ineffective or require time-consuming repetition. Therefore, care facilities are forced to stock many different size balloon catheters and medical care providers must spend time carefully selecting the properly sized balloon catheter. What is needed is a variable length balloon catheter. Therefore, it would be desirable to provide an improved structure for such a balloon catheter having a retractable sheath that can quickly and easily adjust the length of the portion of the balloon to be inflated and a locking mechanism for selectively retaining said sheath in the desired position for use.

Due to internal pressure, shape, and texture, a partially exposed balloon catheter would have a natural tendency to escape the retractable sheath when inflated. This is much like how a partially protruding watermelon seed has a natural tendency to entirely escape a person's lips, due to the watermelon seed's shape and texture. Here, the same phenomenon applies to a partially exposed balloon catheter from a sheath, though the situation is further complicated by the internal pressure of the balloon. Therefore, it would be desirable to additionally provide a mechanical stop in conjunction with the device where said mechanical stop serves to prevent the balloon from entirely escaping the retractable sheath when the balloon is inflated. Further, the mechanical stop can be used to adjust the effective area of the balloon catheter by controlling the length of the balloon permitted to extend from the sheath.

Recently, medical practitioners have realized that drug coatings may be applied to the outer surface of the intravascular balloons to increase the effectiveness of medical treatment. These so called "drug coated balloons" are exposed to blood and other intravascular fluids upon being inserted into a person's vascular system. This may result in the premature activation, elution, dilution, and loss of the drug coating on the way to the treatment zone. Therefore, it would be desirable to provide a watertight seal between the balloon and the sheath such that the medicated balloon is not exposed to intravascular fluids until the balloon reaches the treatment zone.

WO2014/142801 discloses an adjustable balloon catheter including an inner tubular member comprising an inflation lumen and a fluid lumen defined therein. An expandable member is coupled to the distal end portion and has an inner chamber defined therein in fluid communication with the inflation lumen, the expandable member transitionable between a deflated and inflated configuration. The expandable member further has an exterior surface, the exterior surface having at least one pore structure defined therein in fluid communication with the fluid lumen. An outer tubular member is movable relative to the inner tubular member, the outer tubular member being selectively positioned between an extended position and a retracted position to define an exposed length of the working length of the expandable member. The present

invention is directed to an improved structure for an intravascular balloon catheter, as set out in claim 1, having a retractable sheath that can quickly and easily adjust the length of the inflated portion of the balloon and a locking mechanism for selectively retaining the sheath in the desired position for use. The inner member may comprise a series of indentations or protrusion that engage the clamp such that the user may selectively control the location of the retractable sheath relative to the balloon.

The assembly includes a mechanical stop that is configured to selectively secure the balloon and prevents it from entirely escaping the sheath when the balloon is inflated. The assembly may comprise a series of graduated markers, such as radiopaque markers, that indicate the position of the balloon relative to the retractable sheath. The balloon may further comprise a strength collar, such as an annulus, located on the distal end of the retractable sheath.

Additionally, the balloon and the sheath form a substantially watertight engagement with one another. This seal may prevent the medicated coating from being prematurely removed, eluted, or activated prior to the assembly being properly located at the treatment site. In exemplary embodiments of the present invention, the assembly may further comprise a cap that provides a substantially watertight engagement between said cap and the sheath when the balloon is in a retracted position such that the cap abuts the sheath. The balloon may further comprise a lubricious coating between the outer surface of the balloon and the inner surface of the sheath which allows the balloon to move relative to the sheath without disturbing the medicated coating.

Various aspects of this invention will become apparent to those skilled in the art from the following detailed description of the preferred embodiments, when read in light of the accompanying drawings.

### BRIEF DESCRIPTION OF THE DRAWINGS

In addition to the features mentioned above, other aspects of the present invention will be readily apparent from the following descriptions of the drawings and exemplary embodiments, wherein like reference numerals across the several views refer to identical or equivalent features, and wherein:
**Figure 1** is a side elevation view of a first end of a balloon catheter assembly;
**Figure 2** is an enlarged sectional elevation view of a second end of the balloon catheter assembly illustrated in figure 1;
**Figure 3** is a perspective view of a clamp of the balloon catheter assembly illustrated in figure 1 ;
**Figure 4** is an enlarged sectional elevation view showing the clamp of the balloon catheter assembly illustrated in figure 1 in a locked condition;
**Figure 5** is an enlarged sectional elevation view similar to figure 4 showing the clamp of the balloon catheter assembly in an unlocked condition;
**Figure 6** is a further enlarged sectional elevation view of a second end of a balloon catheter assembly;
**Figure 7** is an enlarged sectional elevation view similar to figure 4, wherein a series of spaced indentations/protrusions are provided on one side of the movable sheath;
**Figure 8** is an enlarged sectional elevation view similar to figure 7, wherein a series of annular spaced indentations/protrusions are provided about the circumference of the movable sheath;
**Figure 9** is a sectional elevation view similar to figure 2 showing the end of the sheath in sealing engagement with a tapered insertion tip and indicating Detail A;
**Figure 10** is an enlarged view of the tapered insertion tip illustrated in Detail A of figure 9;
**Figure 11** is an enlarged view similar to figure 10 showing the sheath partially retracted to expose the balloon;
**Figure 12** is a sectional elevation view similar to figure 9 showing an example of a mechanical stop;
**Figure 13** is a sectional elevation view similar to figure 12 showing the embodiment according to the present invention of the mechanical stop;
**Figure 14** is a sectional elevation view similar to figure 12 showing another example the mechanical stop;
**Figure 15** is a sectional elevation view similar to figure 12 showing another example of the mechanical stop;
**Figure 16** is a sectional elevation view similar to figure 12 showing another example of the mechanical stop; and
**Figure 17** is a sectional elevation view similar to figure 12 showing another example of the mechanical stop.

### DETAILED DESCRIPTION OF EXEMPLARY EMBODIMENT(S)

Various embodiments of the present invention will now be described in detail with reference to the accompanying drawings. In the following description, specific details such as detailed configuration and components are merely provided to assist the overall understanding of these embodiments of the present invention. Therefore, it should be apparent to those skilled in the art that various changes and modifications of the embodiments described herein can be made without departing from the scope of the present invention. In addition, descriptions of well-known functions and constructions are omitted for clarity and conciseness.

The invention is described more fully hereinafter with reference to the accompanying drawings, in which exemplary embodiments of the invention are shown. This invention may, however, be embodied in many different forms and should not be construed as limited to the exemplary embodiments set forth herein. Rather, these embodiments are provided so that this disclosure will be thorough and complete, and will fully convey the scope of the invention to those skilled in the art. In the drawings, the size and relative sizes of layers and regions may be exaggerated for clarity.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/ or components, but do not preclude the presence or addition of one or more other features, integers, steps, operations, elements, components, and/or groups thereof.

Embodiments of the invention are described herein with reference to illustrations that are schematic illustrations of idealized embodiments (and intermediate structures) of the invention. As such, variations from the shapes of the illustrations as a result, for example, of manufacturing techniques and/or tolerances, are to be expected. Thus, embodiments of the invention should not be construed as limited to the particular shapes of regions illustrated herein but are to include deviations in shapes that result, for example, from manufacturing.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

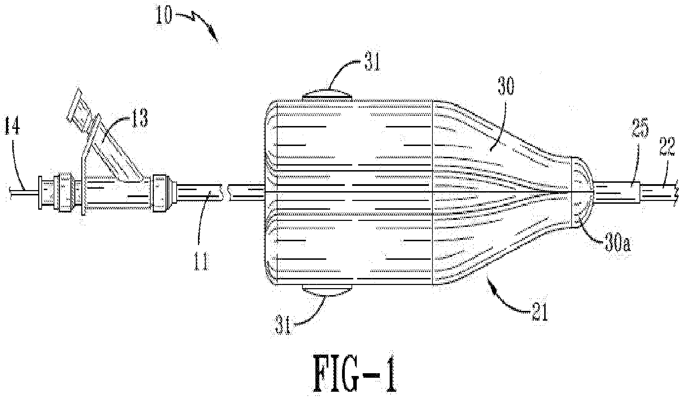

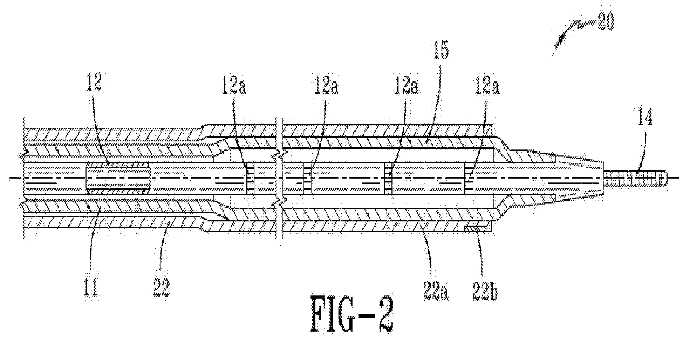

**FIGURE 1** and **FIGURE 2** illustrate a balloon catheter assembly, indicated generally at 10. The balloon catheter assembly is comprised of a first end, illustrated in figure 1, and a second end, illustrated in figure 2. The balloon catheter assembly 10 has an elongated shaft portion that includes an outer member 11 and an inner member 12. The outer member 11 and the inner member 12 may both be tubular in shape, although such is not required. The inner member 12 is disposed within the outer member 11. Thus, an annular outer lumen is defined between the inner surface of the outer tubular member 11 and the outer surface of the inner tubular member 12. Similarly, a cylindrical inner lumen is defined within the inner tubular member 12. The purpose for the outer and inner lumens will be explained below.

In exemplary embodiments of the present invention, one or more radiopaque markers 12a may be provided on the inner tubular member 12. The radiopaque markers 12a are conventional in the art and are provided to facilitate the determination of the location of the inner tubular member 12 during a procedure using conventional fluoroscopy techniques. The radiopaque markers 12a may be spaced in regular intervals. Further, one or more radiopaque markers 22b may be provided on the expanded portion 22a of the sheath 22 to facilitate the determination of the location of the sheath 22 using conventional fluoroscopy techniques during use. The radiopaque markers 12a and 22b may be monitored by the user to determine how far the inner member 12a, and thus the balloon 15, is extended from the sheath 22 thereby permitting the user to monitor how much of the balloon 15 is exposed for treatment of the affected area.

An adapter 13 may be connected to a first end of the elongated shaft portion of the balloon catheter assembly 10. The adapter 13 is conventional in the art and is provided to facilitate access to both the outer and inner lumens during use of the balloon catheter assembly 10. For example, a guide wire 14 can be inserted into the inner lumen via the adapter 13 and through a second end of the elongated shaft portion of the balloon catheter assembly 10, as shown in figure 2. The guide wire 14 is conventional in the art and is provided to facilitate the positioning of the balloon catheter assembly 10 at a desired location for use, such as within a blood vessel as described above.

The outer tubular member 11 includes an inflatable balloon 15. The balloon 15 is conventional in the art and is connected to the outer tubular member 11 such that at least a portion of the inflatable balloon 15 extends about an end portion of the inner tubular member 12. The interior of the balloon 15 communicates with the outer lumen of the balloon catheter assembly 10. As a result, an inflation fluid can be selectively introduced into the adapter 13 and through the outer lumen to the interior of the balloon 15, causing it to inflate in a known manner. Additionally, the interior of the balloon 15 can be vented through the outer lumen and the adapter 13 to the atmosphere, allowing it to deflate after use.

The balloon catheter assembly 10 includes an adjustable sheath assembly, indicated generally at 20. As will be explained in detail below, the adjustable sheath assembly 20 is provided to selectively define a portion of the balloon 15 that is desired to be inflated during use. To accomplish this, the balloon catheter assembly 10 includes a clamp, indicated generally at 21, and a sheath 22. The clamp 21 is disposed about the outer member 11 adjacent to the adapter 13 and the first end of the elongated shaft portion of the balloon catheter assembly 10. The structure and operation of the clamp 21 will be explained in detail below.

The sheath 22 may extend from the clamp 21 about the outer member 11 toward the second end of the elongated shaft portion of the balloon catheter assembly 10. In the illustrated embodiment, the sheath 22 includes an expanded portion 22a that is located adjacent to the second end of the elongated shaft portion of the balloon catheter assembly 10. One or more optional radiopaque markers 22b may be provided on the expanded portion 22a of the sheath 22 to facilitate the determination of the location of the sheath 22 using conventional fluoroscopy techniques during use. Also, the expanded portion 22a of the sheath 22 may have an elastically expandable distal tip provided thereon that is expanded in a conventional manner when the balloon 15 is inflated. The structure and operation of the optional elastically expandable distal tip will be explained in detail below.

Treatments to various size vascular zones of attention may be provided utilizing the present invention. To perform such treatments, a user may first provide an intravascular catheter device consistent with the present invention. Next, the user may direct the catheter to a pre-determined zone of attention within a person's vascular system. Then the user may move the sheath relative to the balloon so to selectively expose a portion of the balloon for inflation. The user may next temporarily secure the sheath relative to the balloon. The user may then inflate the balloon. In exemplary embodiments of the present invention, these steps may include providing a medical treatment device, such as but not limited to a stent, in conjunction with the present invention. The medical treatment device may be deployed when the balloon is inflated, or it may be deployed as another step. In either case, the balloon may then be deflated and removed.

The present disclosure may also provide a method (not claimed) for minimizing or all together eliminating premature activation, elution, dilution, or removal of a drug coating on the balloon and the medical treatment device. To do so, first the user may provide an intravascular catheter device consistent with the present invention. Next, the user may direct the catheter to the pre-determined zone of attention within the person's vascular system. Then the user may move the sheath relative to the balloon so to selectively expose a portion of the drug coated balloon or the medical treatment device for activation. The balloon may be inflated to place the outer surface of the balloon and the medical treatment device in contact with the surrounding blood vessel wall. The balloon may be deflated and removed. It is notable that while the aforementioned method is described with the medical treatment device, such is not required.

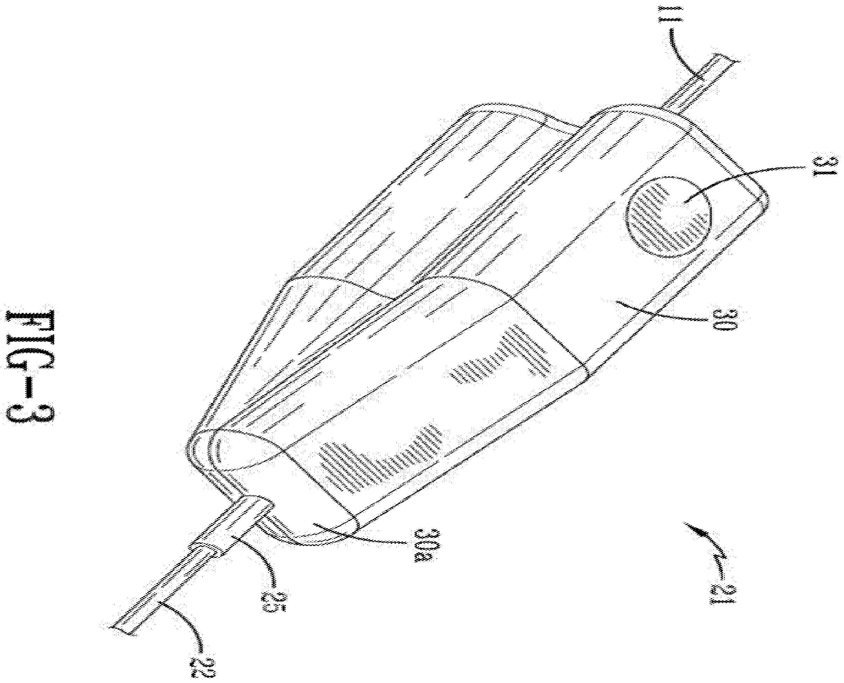

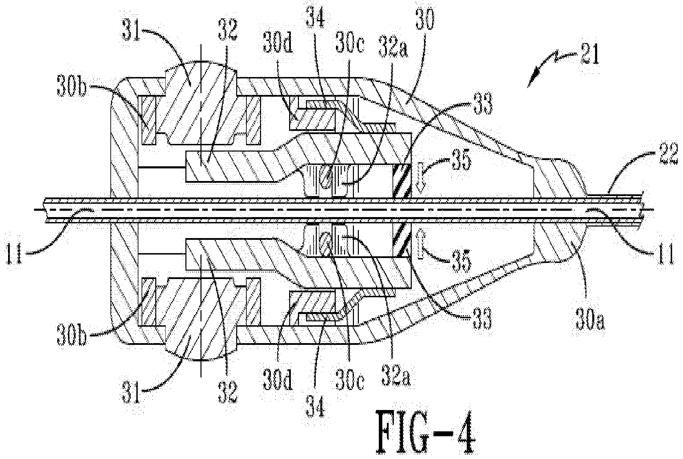

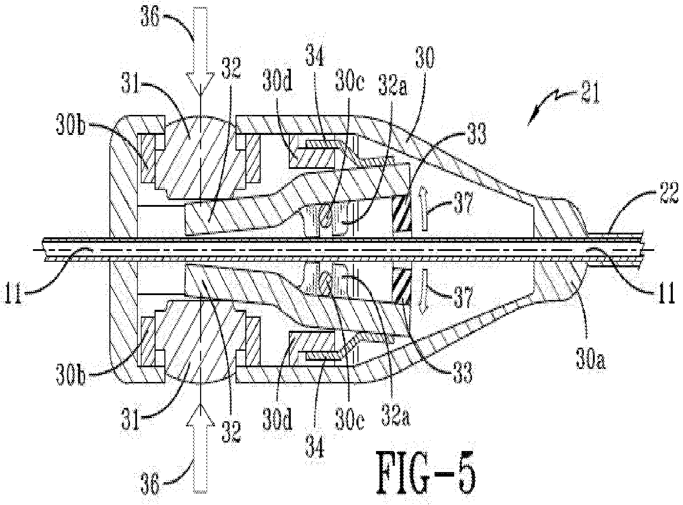

**FIGURE 3** illustrates the external structure of an exemplary embodiment of the clamp 21. As further illustrated in figure 4 and figure 5, the clamp 21 includes a housing 30 through which the outer member 11 of the elongated shaft portion of the balloon catheter assembly 10 extends. The housing 30 includes a push button 31 and a tip 30a. The tip 30a may include a protective member 25 that permits the passage of the sheath 22. In exemplary embodiments of the present invention, the sheath 22 is connected directly to the tip 30a. Thus, the housing 30 of the clamp 21 is connected to the sheath 22 for movement therewith, as will be explained in detail below.

**FIGURE 4** and **FIGURE 5** illustrate the internal structure of an embodiment of the clamp 21. The housing 30 of the clamp 21 also includes a pair of button supports 30b, within which respective manually operable push buttons 31 are supported for inward and outward movement relative to the housing 30. In the illustrated embodiment, each of the button supports 30b is generally hollow and cylindrical in shape, and each of the push buttons 31 is generally cylindrical in shape. However, the button supports 30b and the push buttons 31 may have any desired shape or combination of shapes. The purpose for the push buttons 31 will be explained below.

The housing 30 of the clamp 21 further includes a pair of actuator arm supports 30c, upon which respective actuator arms 32 are supported for pivoting movement relative to the housing 30. In the illustrated embodiment, each of the actuator arm supports 30c is a generally cylindrical protrusion that extends inwardly within the housing 30. However, the actuator arm supports 30c may have any desired shape or combination of shapes. Each of the illustrated actuator arms 32 is provided with a pair of legs 32a that define a pocket. The actuator arm supports 30c of the actuator arms 32 are respectively received within the pockets defined by the pairs of legs 32a of the actuator arms 32. Thus, as will be explained in detail below, the actuator arms 32 are respectively supported on the actuator arm supports 30c of the housing 30 for independent pivoting movement relative thereto.

The first ends of the actuator arms 32 are respectively disposed adjacent to the inner ends of the push buttons 31. The actuator arms 32 also include respective second ends that are disposed remotely from the first ends. In the illustrated embodiment, respective engagement pads 33 are secured to the inner surfaces of the second ends of the actuator arms 32. The engagement pads 33 may be formed from any desired material or combination of materials and may, if desired, be omitted entirely. However, it is preferred that the engagements pads 33 be formed from a material having a relatively high coefficient of friction, such as rubber. The purpose for the engagement pads 33 will be explained below.

Lastly, the housing 30 of the clamp 21 may include a pair of springs 34 that independently bias the two actuator arms 32 toward each other. In the illustrated embodiment, each of the springs 34 is formed from a flat piece of resilient material, such as a metallic material, that has been deformed to achieve a desired shape and bias. However, the springs 34 may be formed from any desired material or combination of materials and take any shape. The springs 34 may have first ends that are received within respective spring supports 30d provided on the inner surface of the housing 30. The springs 34 also may have second ends that bear inwardly upon respective outer surfaces of the second ends of the actuator arms 32. The purpose for the springs 34 will be explained below.

**FIGURE 4** illustrates the clamp 21 of the balloon catheter assembly 10 in a locked condition. In this locked condition, the second ends of the springs 34 bear inwardly upon respective outer surfaces of the second ends of the actuator arms 32. As a result, the engagements pads 33 are urged inwardly toward one another, as shown at 35, so as to engage and may exert a force on opposed portions of the outer surface of the outer member 11 of the balloon catheter assembly 10, which extends through the interior of the housing 30 of the clamp 21. As mentioned above, the engagements pads 33 are preferably formed from a material having a relatively high coefficient of friction. Thus, when the clamp 21 of the balloon catheter assembly 10 is in the locked condition, the outer member 11 of the balloon catheter assembly 10 is hindered or wholly prevented from moving axially relative to the housing 30 of the clamp 21. As mentioned above, the housing 30 of the clamp 21 is connected at the tip 30a to the sheath 22 for movement therewith. Accordingly, when the clamp 21 of the balloon catheter assembly 10 is in the locked condition, the outer member 11 of the balloon catheter assembly 10 is also prevented from moving axially relative to the sheath 22.

**FIGURE 5** illustrates the clamp 21 of the balloon catheter assembly 10 in an unlocked condition. To achieve this unlocked condition, a user of the balloon catheter assembly 10 may apply inwardly directed forces, as shown at 36, against the manually operable push buttons 31. These forces overcome the forces exerted by the springs 34 and cause the actuator arms 32 to pivot such that the engagements pads 33 are moved out of engagement, as shown at 37, with the opposed portions of the outer surface of the outer member 11 of the balloon catheter assembly 10. Thus, when the clamp 21 of the balloon catheter assembly 10 is in the unlocked condition, the outer member 11 of the balloon catheter assembly 10 is permitted to move axially relative to the housing 30 of the clamp 21. As mentioned above, as the housing 30 of the clamp 21 may be connected at the tip 30a to the sheath 22 for movement therewith, when the clamp 21 of the balloon catheter assembly 10 is in the unlocked condition, the outer member 11 of the balloon catheter assembly 10 is also permitted to move axially relative to the sheath 22.

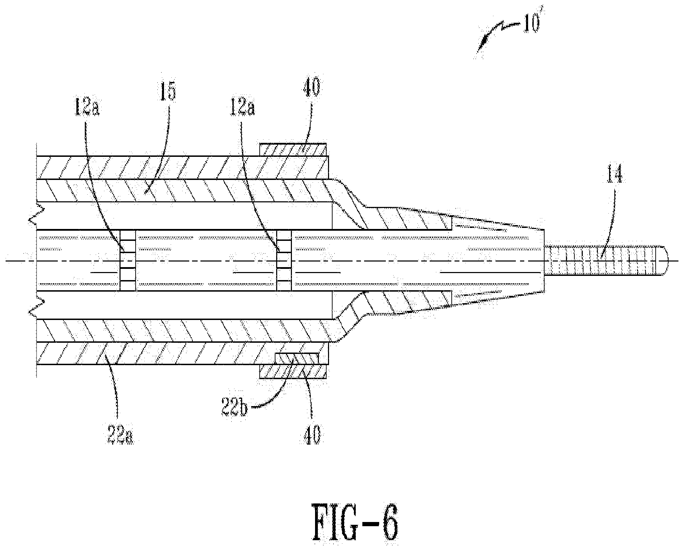

FIGURE 6 illustrates a second end of the balloon catheter assembly, indicated generally at 10', in accordance with this invention. Like reference numbers are used to indicate components that are the same as described above. As shown therein, the second end of the modified balloon catheter assembly 10' has a collar 40 provided thereon. The purpose of the collar 40 is to prevent the sheath 22 from splitting or otherwise being deformed or damaged as a result of the pressure force exerted by the balloon 15 when it is inflated. The illustrated collar 40 is tubular in shape and extends completely about the end of the sheath 22 where the balloon 15 is located. However, it will be appreciated that the collar 40 may have any desired shape and may be located at any desired position on the sheath 22 or cover any desired portion of the sheath 22. The collar 40 may be formed from any desired material, such as a metallic material, a high strength aramid fiber material (such as is commercially available under the Kevlar^{®} brand name), a high durometer plastic material, or the like. If desired, the collar 40 may be formed from a radiopaque material, allowing it to function as a marker for the distal tip of the sheath 22. If desired, the distal tip of the sheath 22 may be provided with a smooth edge (not shown) so as not to present a sharp edge toward the balloon 15.

The tip of the balloon 15 may fit snugly into the end of the sheath 22 so as to provide a watertight engagement therebetween. Such a watertight engagement would maintain the integrity of the interior compartment of the balloon catheter assembly 10 during its insertion within a blood vessel or other portion of a body and during travel to the treatment site. This may be utilized to maintain a dry interior. Maintaining the interior compartment of the balloon catheter assembly 10 dry facilitates the use of conventional drug-coated balloons, stents, and other devices. The coatings provided on such devices are, in some instances, activated by contact with blood and water. In known catheter assemblies, a significant amount of the drugs provided on the balloons, stents, and other devices can be eluted during the initial insertion of the catheter assembly through blood vessels having flowing blood. The watertight engagement of this invention allows for minimal elution of the drugs during the initial insertion of the balloon catheter assembly 10 and maximal delivery of the drugs to the desired site. As will be explained in greater detail in subsequent figures, a sealed engagement is obtained by the use of a tapered insertion tip 60. A lubricious coating may be provided between the sheath 22 and the outer surface of the balloon 15, such that the lubricious coating may reduce or eliminate disturbance to the drug coating when the balloon 15 is moved relative to the sheath 22.

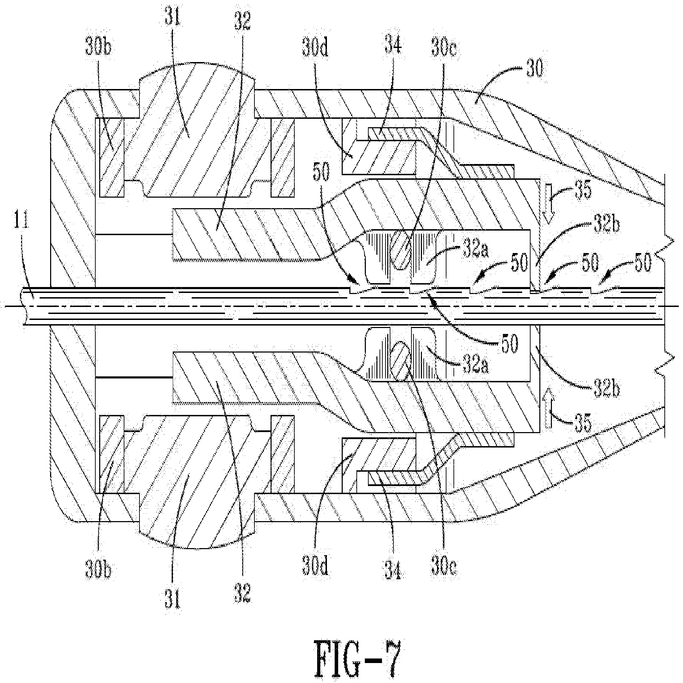

**FIGURE 7** illustrates one side of the outer member 11 provided with a plurality of spaced indentations or protrusions, indicated generally at 50. The indentations or protrusions 50 may have any desired shape or size (or combination of shapes and sizes) and may be provided at any desired location(s) on the one side of the outer member 11. Any number of indentations or protrusions 50 is contemplated. The indentations or protrusions 50 are adapted to cooperate with one or more inwardly extending portions 32b provided on either or both of the actuator arms 32. Thus, when the clamp 21 of the balloon catheter assembly 10 is in the locked condition, the inwardly extending portions 32b provided on either or both of the actuator arms 32 engage one or more of the indentations or protrusions 50. As a result, the outer member 11 of the balloon catheter assembly 10 is prevented from moving axially relative to the housing 30 of the clamp 21. Conversely, when the clamp 21 of the balloon catheter assembly 10 is in the unlocked condition, the inwardly extending portions 32b provided on either or both of the actuator arms 32 do not engage one or more of the indentations or protrusions 50. As a result, the outer member 11 of the balloon catheter assembly 10 is permitted to move axially relative to the housing 30 of the clamp 21.

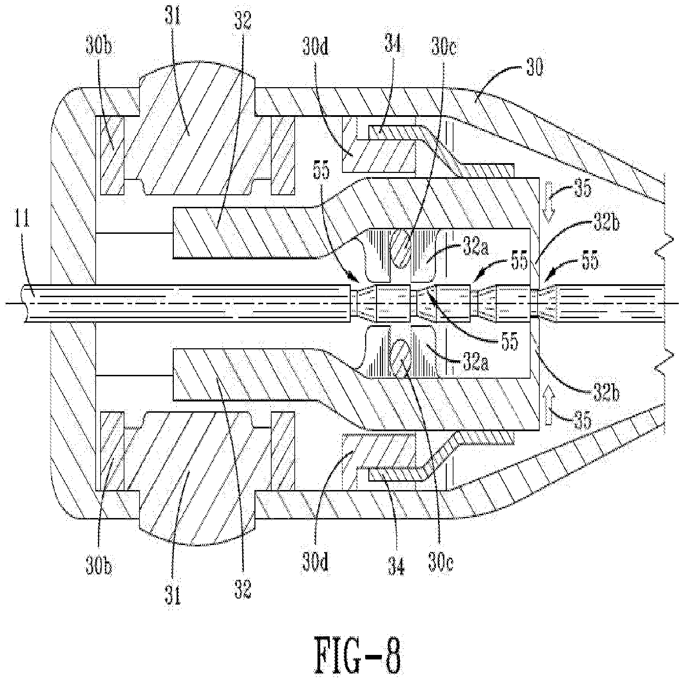

**FIGURE 8** illustrates a series of annular spaced indentations or protrusions, indicated generally at 55. The indentations or protrusions 55 may have any desired shape or size (or combination of shapes and sizes) and may be provided at any desired location(s) on the outer member 11. Any number of indentations or protrusions 55 is contemplated. The indentations or protrusions 55 are adapted to cooperate with one or more of the inwardly extending portions 32b provided on either or both of the actuator arms 32. Thus, when the clamp 21 of the balloon catheter assembly 10 is in the locked condition, the inwardly extending portions 32b provided on either or both of the actuator arms 32 engage one or more of the indentations or protrusions 55. As a result, the outer member 11 of the balloon catheter assembly 10 is prevented from moving axially relative to the housing 30 of the clamp 21. Conversely, when the clamp 21 of the balloon catheter assembly 10 is in the unlocked condition, the inwardly extending portions 32b provided on either or both of the actuator arms 32 do not engage one or more of the indentations or protrusions 55. As a result, the outer member 11 of the balloon catheter assembly 10 is permitted to move axially relative to the housing 30 of the clamp 21.

The annular spaced indentations or protrusions 55 may be a series of identical annular spaced indentations or protrusions 55. Each section of the annular spaced indentations or protrusions 55 may be defined by a tubular section with an outer diameter smaller than the outer diameter of the outer member 11, a conical section with an initial outer diameter equal to the smaller outer diameter of the previous section, transitioning to an outer diameter substantially equal to the outer diameter of the outer member 11, and finally, a second tubular section with an outer diameter substantially equal to the outer diameter of the outer member. This design allows for the inwardly extending portions 32b to ratchet as each section of the annular spaced indentation or protrusion 55 passes the inwardly extending portions 32b. This may provide the user with tactile and audible feedback as each section passes through the inwardly extending portion 32b. In the illustrated embodiment this would prevent the clamp 21 from moving distally relative to the balloon catheter assembly 10. It is contemplated that the annular spaced indentations or protrusions 55 may be reversed such that the indentations or protrusions 55 prevent the clamp 21 from moving proximally relative to the balloon catheter assembly 10.

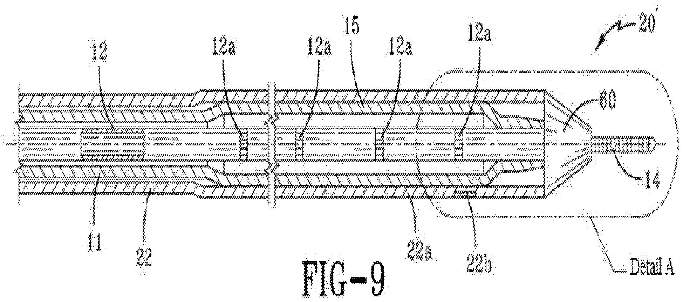

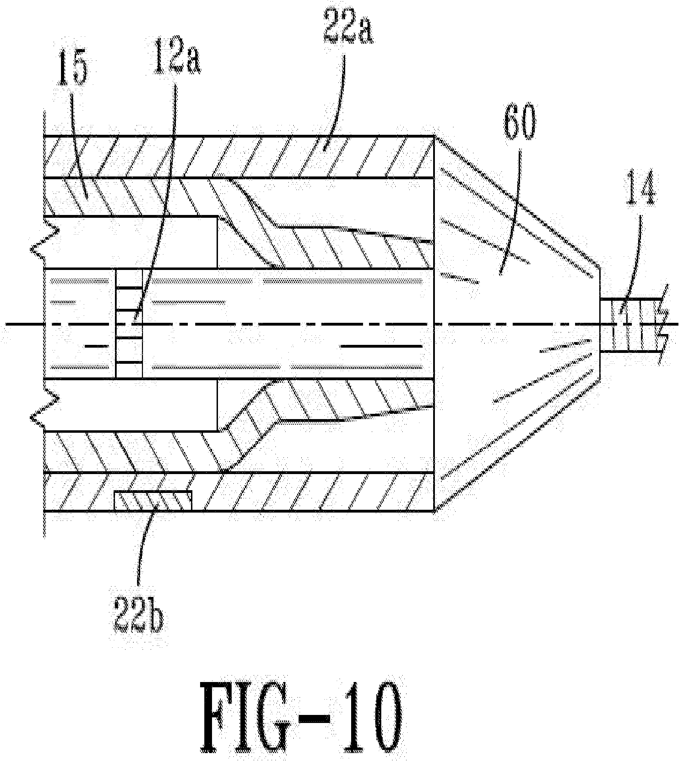

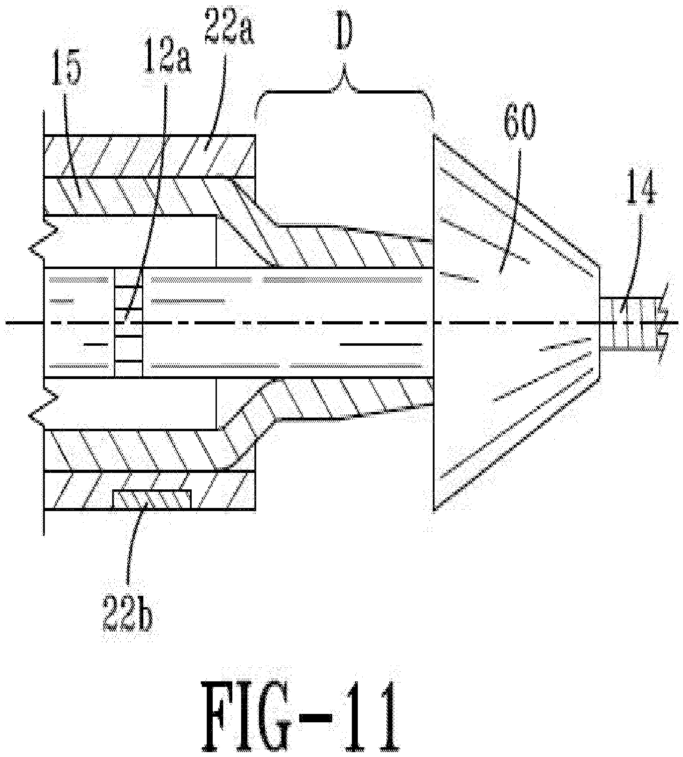

**FIGURE 9****,** **FIGURE 10****,** and **FIGURE 11** illustrate a modified end of the adjustable sheath assembly 20' with the tapered insertion tip 60 according to the invention. In figure 9 and figure 10, the end of the adjustable sheath assembly 20' is in sealing engagement with the tapered insertion tip 60. Figure 11 is similar to figure 10, but shows the end of the adjustable sheath assembly 20' partially retracted to expose the balloon 15 in the manner described above. One or more radiopaque markers (not shown) may be placed on the tapered insertion tip 60 and monitored using conventional fluoroscopy techniques such that the user may determine the distance, shown in figure 11 as "D", between the sheath 22a and the tapered insertion tip 60. This also permits the user to determine the exposed length of the balloon 15.

The sheath 22 and tapered insertion tip 60 may protect the drug coating or drug coated devices such that the drug coating is not activated, eluted, diluted, or removed when the balloon catheter assembly 10 is placed into the blood vessel. In exemplary embodiments of the present invention, the balloon 15 alone may provide a sealing engagement with the sheath 22. The balloon catheter assembly 10 may additionally comprise a lubricious coating provided between the sheath 22 and the balloon 15 to facilitate the movement of the balloon 15 relative to the sheath 22 by reducing or eliminating disturbance to the drug coating. In exemplary embodiments of the present invention, the lubricious coating may additionally or alternatively be combined with or placed over the top of the medicated coating.

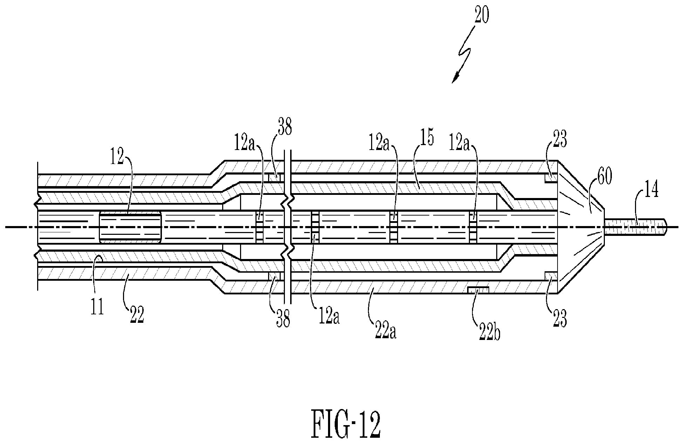

**FIGURE 12** illustrates the adjustable sheath assembly 20 of the balloon catheter assembly 10, further comprising a mechanical stop which is not part of the invention. The mechanical stop may comprise an annulus 23 and a block 38. A sheath lumen may be defined as the opening between the outer surface of balloon 15 and the expanded portion 22a. In exemplary embodiments of the present invention, there may be no sheath lumen, as the sheath 22 substantially fits the curvature of the balloon 15. The annulus 23 may be located on the distal end of the expanded portion 22a. The annulus 23 may comprise an annual member located on the inner surface of the expanded portion 22a having a thickness such that it protrudes inwardly, thereby restricting the sheath lumen. The stop 38 may comprise a corresponding annual member 38 located on the outer diameter of the balloon 15, though any shape capable of frictionally engaging the annulus 23 is contemplated. The outer dimension of the stop 38 has an outer diameter larger that the lumen created by the annulus 23. In such a case, the outer diameter of the corresponding stop 38 is configured such that it frictionally engages annulus 23, thereby preventing the balloon 15 from extending beyond the annulus 23.

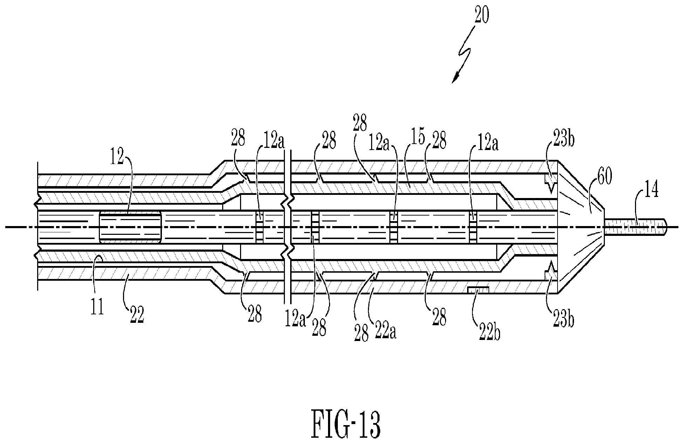

**FIGURE 13** illustrates the embodiment of the present invention in which the assembly 10 further comprises one or more ridges 28 located on the outer surface of the balloon 15 that extend vertically therefrom. The mechanical stop comprises an annular shaped pointed member 23b that protrudes inwardly from the inner surface of the distal end of the expanded portion 22a, thereby restricting the sheath lumen, and the ridges 28. Further, the pointed shape of the pointed member 23b may be complementary to the shape of each of the ridges 28. As the balloon 15 is advanced relative to the sheath 22, the pointed member 23b selectively secures the balloon 15 by frictionally engaging each of the ridges 28. In exemplary embodiments, the ridges 28 are formed as part of the balloon 15. Any number of ridges 28 is contemplated. Additionally, the ridges 28 may be equally spaced apart along balloon 15, though any spacing is contemplated. The series of ridges 28 may be comprised of a sufficiently rigid material such that each ridge 28 may not pass through the lumen created by the pointed member 23b without the user exerting a force on the assembly 10 such that the ridge 28 or the pointed member 23b may be sufficiently deformed to pass though the lumen created by pointed member 23b. This force may be exerted by the user directly on the outer member 11. Alternatively, this force may be exerted by the user on the outer member by use of the clamp 21.

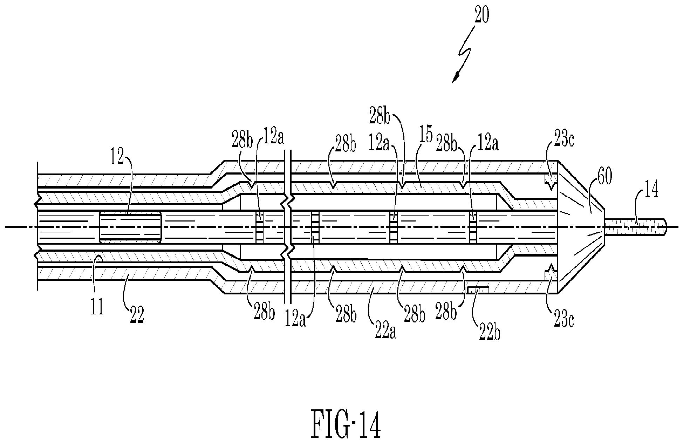

**FIGURE 14** illustrates another example which is not part of the invention wherein the balloon 15 comprises a series of valleys 28b located on the outer diameter of the balloon 15. The valleys 28b reduce the thickness of the balloon 15 such that outer diameter of the balloon 15 is reduced. Any number of valleys 28b is contemplated. Said valleys 28b may be equally spaced apart along the balloon 15, though any spacing is contemplated. A pointed member 23c may selectively secure movement of the balloon 15 relative to the sheath 22 by frictionally engaging each of the valleys 28b as they move through the lumen created by the pointed member 23c. The shape of the pointed member 23b may be formed complementary to the void created by each of the valleys 28b. The pointed member 23c may be comprised of a sufficiently flexible material such that each of the valleys 28b may not pass through the lumen created by the pointed member 23c until the user exerts force on the assembly such that the pointed member 23c or the balloon 15 is sufficiently deformed to permit each of the series of valleys 28b to pass through the lumen created by the pointed member 23c. This force may be exerted by the user directly on the outer member 11. Alternatively, this force may be exerted by the user on the outer member by use of the clamp 21.

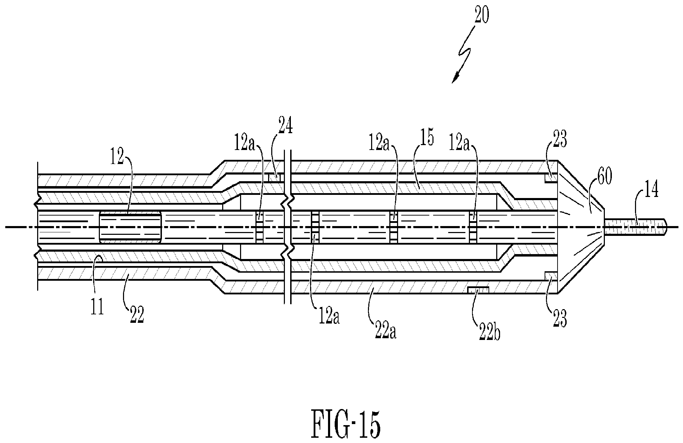

**FIGURE 15** illustrates another example which is not part of the present invention wherein the mechanical stop comprises a block 24, which may be attached to the balloon 15. The block 24 may be attached to the outer diameter of the balloon 15. The block 24 may be in the shape of a "T" such that the vertical section of the "T" extends vertically from the outer surface of balloon 15 and the horizontal portion of the "T" is curved and secured to the outer surface of the balloon 15, though any shape is contemplated. As the balloon 15 is extended from the expanded portion 22a, the block 24 frictionally engages the mechanical stop 23. In other examples, the block of any shape may be used in place of the block 24.

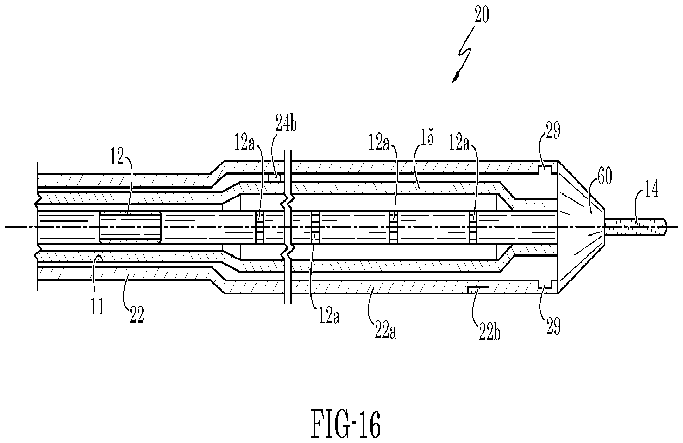

**FIGURE 16** illustrates another example which is not part of the invention of the mechanical stop comprising a circumferential slot 29 located on the inner surface of the expanded portion 22a. The slot 29 may be configured to accommodate and frictionally engage a collapsible block 24b such that as the balloon 15 is extended from the expanded portion 22a, the collapsible block 24b expands and enters the slot 29, thereby preventing the balloon 15 from further extending relative to the expanded portion 22a. In other examples, a collapsible orthotope, column, "T", or other shape collapsible device may be used. The collapsible block 24b, or the portion of the collapsible block 24b extending into the slot 29, may be spring loaded or otherwise biased into an expanded position.

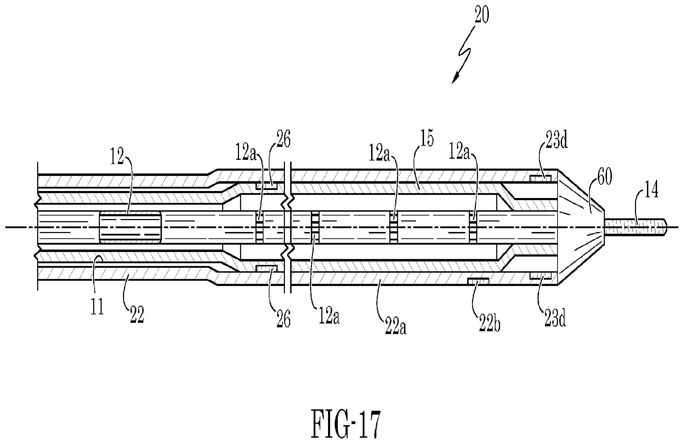

**FIGURE 17** illustrates another example which is not part of the invention in which the mechanical stop comprises a first and second portion 26 and 23d. The first and second portion 26 and 23d may be located on the outer surface of the balloon 15 and the inner surface of the sheath 22, respectively, and may comprise a surface or a coating with a relatively high coefficient of friction. The remainder of the balloon 15 and the sheath 22 may comprise a surface or a coating with a relatively low coefficient of friction such as a polymer, silicone, and/or further comprising a lubricant is contemplated. The first and second portions 26 and 23d may comprise a rubber, polymer, or the like. Further, the first and second portions 26 and 23d may be comprised of a surface texture to increase the coefficient of frictions such as bumps or the like is contemplated. The first portion 26 may be located towards the proximal end of the balloon 15 relative to the clamp 21, though any location is contemplated. The second portion 23d may be located towards the distal end of the sheath 22 relative to the clamp 21, though any location is contemplated. The balloon 15 may extend relative to the sheath 22 until the first portion 26 encounters and frictionally engages the second portion 23d, thereby preventing the balloon 15 from traveling beyond.

It is notable that the mechanical stop described herein may be formed separately and affixed to the balloon or other components, or may be integrally formed with the balloon or other component.

## Claims

1. An intravascular balloon catheter device (10) comprising:
an inner member (12);
an outer member (11) disposed about the inner member (12) and comprising a
balloon (15) configured for inflation to place the outer surface of the balloon (15) in contact with an inner surface of the surrounding blood vessel wall, said balloon (15) having a proximal end and a distal end, wherein the proximal end of the balloon (15) is connected to the outer member (11) and the distal end of the balloon (15) is connected to the inner member (12);
a sheath (22) disposed about the outer member (11) and adapted for movement
relative to the balloon (15) so as to selectively expose a second portion of the balloon (15) for inflation;
a tapered insertion tip (30a) disposed about an end of the outer member (11) and
configured to provide a substantially watertight engagement between said tip (30a) and said sheath (22) when said tip (30a) is abutted to said sheath (22);
a clamp (21) configured to selectively secure the sheath (22) at a desired
position relative to the balloon (15) such that a first portion of the balloon (15) remains located within the sheath (22); and
a mechanical stop configured to prevent the balloon (15) from fully escaping the
sheath (22) when the balloon (15) is inflated;
wherein the mechanical stop comprises at least one ridge (28) integrally formed
with and located on an outer surface of the balloon (15) and at least one corresponding ridge (23b) located on an inner surface of the sheath (22);
wherein said ridges (28, 23b) are configured to frictionally engage one another as
the balloon (15) is moved relative to the sheath (22);
wherein the sheath (22) is configured to prevent the first portion of the balloon
(15) from inflating to a diameter larger than the sheath (22).

2. The device of Claim 1 further comprising:
a series of radiopaque marker bands (12a) located at select intervals on the
outer member (11).

3. The device of Claim 1 further comprising:
a collar (40) provided about an end of the sheath (22) and adapted to provide
structural rigidity to the sheath (22).

4. The device of Claim 1 further comprising:
a drug coating provided on the outer surface of the balloon (15).

5. The device of Claim 4 further comprising:
a lubricious coating provided between the outer surface of the balloon (15) and
the inner surface of the sheath (22).

## Patentansprüche

1. Intravaskuläre Ballonkathetervorrichtung (10), umfassend:
ein inneres Element (12);
ein äußeres Element (11), das um das innere Element (12) angeordnet ist und umfassend einen Ballon (15), der zum Aufblasen konfiguriert ist, um die Außenfläche des Ballons (15) in Kontakt mit einer Innenfläche der umgebenden Blutgefäßwand zu bringen, wobei der Ballon (15) ein proximales Ende und ein distales Ende aufweist, wobei das proximale Ende des Ballons (15) mit dem äußeren Element (11) verbunden ist und das distale Ende des Ballons (15) mit dem inneren Element (12) verbunden ist;
eine Hülle (22), die um das äußere Element (11) angeordnet und für eine Bewegung in Bezug auf den Ballon (15) geeignet ist, um selektiv einen zweiten Abschnitt des Ballons (15) zum Aufblasen freizulegen;
eine sich verjüngende Einführspitze (30a), die um ein Ende des äußeren Elements (11) angeordnet und konfiguriert ist, um einen im Wesentlichen wasserdichten Eingriff zwischen der Spitze (30a) und der Hülle (22) bereitzustellen, wenn die Spitze (30a) an der Hülle (22) anliegt;
eine Klemme (21), die konfiguriert ist, um die Hülle (22) selektiv in einer gewünschten Position in Bezug auf den Ballon (15) zu halten, sodass ein erster Abschnitt des Ballons (15) innerhalb der Hülle (22) verbleibt; und
einen mechanischen Anschlag, der konfiguriert ist, um zu verhindern, dass der Ballon (15) vollständig aus der Hülle (22) austritt, wenn der Ballon (15) aufgeblasen wird;
wobei der mechanische Anschlag mindestens eine Rippe (28), die einstückig mit einer Außenfläche des Ballons (15) gebildet ist und sich darauf befindet, und mindestens eine entsprechende Rippe (23b), die sich auf einer Innenfläche der Hülle (22) befindet, umfasst;
wobei die Rippen (28, 23b) konfiguriert sind, um reibschlüssig einander einzugreifen, wenn der Ballon (15) in Bezug auf die Hülle (22) bewegt wird;
wobei die Hülle (22) konfiguriert ist, um den ersten Abschnitt des Ballons (15) daran zu hindern, sich auf einen größeren Durchmesser als die Hülle (22) aufzublasen.

2. Vorrichtung gemäß Anspruch 1, ferner umfassend:
eine Reihe von röntgendichten Markierungsstreifen (12a), die sich in bestimmten Abständen auf dem äußeren Element (11) befinden.

3. Vorrichtung gemäß Anspruch 1, ferner umfassend:
einen Bund (40), der um ein Ende der Hülle (22) bereitgestellt und geeignet ist, um der Hülle (22) strukturelle Steifigkeit zu verleihen.

4. Vorrichtung gemäß Anspruch 1, ferner umfassend:
eine Arzneimittelbeschichtung, die auf der Außenfläche des Ballons (15) bereitgestellt ist.

5. Vorrichtung gemäß Anspruch 4, ferner umfassend:
eine gleitfähige Beschichtung, die zwischen der Außenfläche des Ballons (15) und der Innenfläche der Hülle (22) bereitgestellt ist.

## Revendications

1. Un dispositif de cathéter à ballonnet intravasculaire (10) comprenant :
un élément interne (12) ;
un élément extérieur (11) disposé autour de l'élément intérieur (12) et comprenant un ballonnet (15) configuré pour être gonflé afin de placer la surface extérieure du ballonnet (15) en contact avec une surface intérieure de la paroi du vaisseau sanguin environnant, ledit ballonnet (15) ayant une extrémité proximale et une extrémité distale, dans lequel l'extrémité proximale du ballonnet (15) est connectée à l'élément extérieur (11) et l'extrémité distale du ballonnet (15) est connectée à l'élément intérieur (12) ;
une gaine (22) disposée autour de l'élément extérieur (11) et adaptée pour se déplacer par rapport au ballonnet (15) de façon à exposer sélectivement une deuxième partie du ballonnet (15) pour le gonflage ;
une pointe d'insertion effilée (30a) disposée autour d'une extrémité de l'élément extérieur (11) et configurée pour fournir un engagement sensiblement étanche entre ladite pointe (30a) et ladite gaine (22) lorsque ladite pointe (30a) est en butée contre ladite gaine (22) ;
une pince (21) configurée pour fixer sélectivement la gaine (22) à une position souhaitée par rapport au ballonnet (15) de sorte qu'une première partie du ballonnet (15) reste située à l'intérieur de la gaine (22) ; et
une butée mécanique configurée pour empêcher le ballonnet (15) de s'échapper complètement de la gaine (22) lorsque le ballonnet (15) est gonflé ; dans lequel la butée mécanique comprend au moins une nervure (28) formée d'un seul tenant avec et située sur une surface extérieure du ballonnet (15) et au moins une nervure correspondante (23b) située sur une surface intérieure de la gaine (22) ;
dans lequel lesdites nervures (28, 23b) sont configurées pour s'engager par friction l'une avec l'autre lorsque le ballonnet (15) est déplacé par rapport à la gaine (22) ;
dans lequel la gaine (22) est configurée pour empêcher la première partie du ballonnet (15) de se gonfler à un diamètre supérieur à celui de la gaine (22).

2. Le dispositif selon la revendication 1 comprenant en outre :
une série de bandes de marquage radio-opaques (12a) situées à des intervalles choisis sur l'élément extérieur (11).

3. Le dispositif selon la revendication 1 comprenant en outre :
un collier (40) prévu autour d'une extrémité de la gaine (22) et adapté pour fournir une rigidité structurelle à la gaine (22).

4. Le dispositif selon la revendication 1 comprenant en outre :
un enrobage de médicament prévu sur la surface extérieure du ballonnet (15).

5. Le dispositif selon la revendication 4 comprenant en outre :
un revêtement lubrifiant prévu entre la surface extérieure du ballonnet (15) et la surface intérieure de la gaine (22).
